# EUROPEAN PATENT APPLICATION

(11) **EP 1 574 518 A2**
(43) Date of publication of application: **14.09.2005**
(21) Application number: 04256100.1
(22) Date of filing: 01.10.2004
(51) Int. Cl.: C07K 14/46, A61K 38/00

(54) **Venom-derived vascular endothelial growth factor-like protein having binding activity specific to vascular endothelial growth factor receptor type 2 and use thereof**

(30) Priority: 02.10.2003 JP 2003344994
(71) Applicant: Nec Soft, LTD., Tokyo (JP); Morita, Takashi, Tokorozawa-shi, Saitama (JP)
(72) Inventor: Kawai, Hisanori, c/o NEC Soft, Ltd., Koto-ku, Tokyo (JP); Yamazaki, Yasuo, c/o NEC Soft, Ltd., Koto-ku, Tokyo (JP); Morita, Takashi, Tokorozawa-shi, Saitama (JP)
(74) Representative: W.P. Thompson & Co.

(57) **Abstract**

An objective of the present invention is to provide a newly isolated vascular endothelial growth factor (VEGF)-like protein having binding activity specific to a vascular endothelial growth factor receptor type 2 (KDR) but exhibiting no binding affinity to a vascular endothelial growth factor receptor type 1 (Flt-1), and thus being excellent in hypotensive effect.

There have been newly purified and isolated a VEGF-like protein from a venom of *Vipera ammodytes ammodytes*: vammin and a VEGF-like protein from a venom of *Daboia russelli russelli:* VR-1 as the VEGF-like protein having binding activity specific to KDR but exhibiting no binding affinity to Flt-1, and thereby being excellent in hypotensive effect, and the amino acid sequences of these both have been analyzed in the present invention.

## Description

### Technical Field

This invention relates to a newly-isolated venom-derived vascular endothelial growth factor (VEGF)-like protein which has binding activity specific to a vascular endothelial growth factor receptor type 2 (VEGF receptor 2; KDR), but does not exhibit any binding affinity to a vascular endothelial growth factor receptor type 1 (VEGF receptor 1; FIt-1), as well as to use therefor in medicine field. More specifically, the present invention relates to a VEGF-like protein isolated from the venom of *Vipera ammodytes ammodytes:* vammin and a VEGF-like protein isolated from the venom of *Daboia russelli russelli:* VR-1, and to use therefor in medicine field, such as their applications for the treatment of an ischemic disease by means of hypotensive effect of these novel venom-derived VEGF-like proteins (vammin and VR-1) through the reception thereof on KDR.

### Background Art

The vascular endothelial growth factor (VEGF-A) plays a predominant role in vasculogenesis and angiogenesis. It is well known that angiogenesis is involved in various diseases such as solid tumor growth, diabetic retinopathy, premature infant retinitis, rheumatoid arthritis and psoriasis, while it plays an important role in physiological events such as wound healing and endometrium formation or luteinization in a female menstrual cycle. VEGF-A is a glycoprotein where its subunits having a molecular weight of 23 kDa are homo-dimerized via a disulfide bond. Growth factors similar to VEGF-A include VEGF-B, VEGF-C, VEGF-D, VEGF-E and placental growth factor (PIGF) (Masashi Shibuya, Masahiko Kurabayashi ed., Experimental Medicine, 20 (extra number), 1070-1269 (2002); Mayumi Ono, Nobuhiko Kuwano, Biology of angiogenesis and cancer, Kyoritsu Shuppan Co. Ltd., 1-97 (2000); and Yasushi Sato ed., Frontier of angiogenesis, Yodosha Co. Ltd., 10-171 (1999)). VEGF binds to VEGFR-1 (fms-like tyrosine kinase-1: Fit-1) and VEGFR-2 (kinase insert domain-containing receptor: KDR) in a vascular endothelial cell with high affinity. It has been suggested that signaling for an endothelial cell growth signal and hypotensive effect due to VEGF-A may be mainly mediated by KDR (Li, B. et al., Hypertension, 39, 1095-1100 (2002)). It is believed that NO produced owing to the signaling via KDR has various physiological effects such as vasorelaxation effect, inhibition of platelet aggregation, antithrombotic effect and antiinflammatory effect and thus fulfils anti-arteriosclerotic function (Masashi Shibuya, Masahiko Kurabayashi ed., Experimental Medicine, 20 (extra number), 1070-1269 (2002)). More recently, it has been reported that FIt-1 stimulates hepatocyte proliferation (Le, J. et al., Science, 299, 890-893 (2003)). When using VEGF, which may induce KDR-mediated hypotensive effect, for treatment of an ischemic disease, there is a problem of side effects such as hypertrophy of the liver due to its binding to FIt-1. There has been reported, as a protein exclusively binding to KDR, VEGF-E derived from Parapox virus and variant proteins that have been produced by modification of VEGF so as to furnish with selectivity to a receptor molecule. It has been, however, described that the variant proteins of VEGF with the specificity to KDR exhibit lower hypotensive effect than the natural VEGF-A, and that VEGF-E is comparable to or rather less effective than VEGF-A in terms of promotion activity of vascular endothelial cell growth and vascular permeability (Gille, H. et al., J. Biol. Chem., 276, 3222-3230 (2001); United States Patent No. 6057428; United States Patent No. 6020473; United States Patent No. 6541008; WO 00/ 63380 pamphlet; WO 09/708313 pamphlet; Ogawa, S. et al., J. Biol. Chem., 273, 31273-31282 (1998); Meyer, M. et al., EMBO J., 18, 363-374 (1999); and.Wise, L. M. et al, Proc. Natl. Acad. Sci. USA, 96, 3071-3076 (1999)).

Meanwhile, molecules having useful physiological activities are contained in a snake venom. A hypotensive factor (HF) has been isolated from the venom of *Vipera aspis aspis.* HF is a protein of which primary structure (amino acid sequence) has homology to that of VEGF (Komori, Y. et al., Toxicon, 28, 359-369 (1990); and Komori, Y. et al., Biochemistry, 38, 11796-11803 (1990)). Furthermore, two VEGF-like molecules, a snake venom VEGF and an increasing capillary permeability protein (ICPP) have been more recently isolated from other snake venoms, respectively. It has been demonstrated that these two venom-derived VEGF-like proteins have vascular permeability and stimulating activity for angiogenesis (Junqueira de Azevedo, I. L. et al., J. Biol. Chem., 276, 39836-39842 (2001); Gasmi, A. et al., Biochem. Biophys. Res. Commun., 268, 69-72 (2000); and Gasmi, A. et al., J. Biol. Chem., 277, 29992-29998 (2002)).

### Disclosure of Invention

As described above, there has been a concern that when a VEGF-like protein inducing the KDR-mediated hypotensive effect is used for treatment of blood pressure elevation associated with an ischemic disease, the protein, if it also has binding affinity to Flt-1, may stimulate hepatocyte proliferation, which causes side effects such as hypertrophy of the liver. There has been, therefore, a need in this art to seek a novel VEGF-like protein which has binding activity specific to KDR but does not exhibit any binding affinity to FIt-1 and, exhibits nitrogen monoxide (NO) dependent strong hypotensive effect via a mechanism of NO synthetase activation mediated through such binding to KDR.

For solving the above problems, an objective of the present invention is to provide a newly isolated vascular endothelial growth factor (VEGF)-like protein being excellent in hypotensive activity which protein has binding activity specific to vascular endothelial growth factor receptor type 2 (VEGF receptor 2; KDR), but does not exhibit any binding affinity to vascular endothelial growth factor receptor type 1 (VEGF receptor 1; FIt-1), as well as use for medicine thereof. In addition, further objectives of the present invention include determining the amino acid sequence of the newly isolated vascular endothelial growth factor (VEGF)-like protein, identifying partial amino acid sequences (sites) that make significant contribution to effective inhibition of binding to FIt-1 as well as to maintaining tight binding affinity to KDR, and then modifying the amino acid sequence in the natural VEGF-like protein to provide a VEGF-like protein variant retaining strong binding activity specific to KDR and strong hypotensive effect which is comparable to that of the natural VEGF-like protein.

By using multi-step chromatography, we have isolated VEGF-like protein from the venom of *Vipera ammodytes ammodytes:* vammin and VEGF-like protein from the venom of *Daboia russelli russelli:* VR-1 as novel vascular endothelial growth factor (VEGF)-like proteins, and have elucidated the full-length primary structures (amino acid sequences) thereof. After studying the three-dimensional structures of these novel venom-derived VEGF-like proteins: vammin and VR-1, we have found that vammin and VR-1 exist as homo-dimers formed by dimerization of a 110 and a 109 amino-acid peptide chains via interchain disulfide bonds, respectively. We have also confirmed the feature that both of the homo-dimer type VEGF-like proteins, vammin and VR-1 bind to KDR (VEGF receptor 2) with high affinity, but do not bind to Flt-1 (VEGF receptor 1) at all, and exhibit nitrogen monoxide-dependant and strong hypotensive effects via a mechanism of NO synthetase activation mediated through their binding to KDR. Thus, based on these findings and further investigation results, we have achieved the completion of the present invention.

Accordingly, the first embodiment of such a novel VEGF-like protein according to the present invention is a vascular endothelial growth factor (VEGF) like protein derived from *Vipera ammodytes ammodytes;* vammin, wherein the vammin protein has binding activity to a vascular endothelial growth factor receptor type 2 (VEGF receptor 2; KDR), but does not exhibit any binding affinity to a vascular endothelial growth factor receptor type 1 (VEGF receptor 1; FIt-1), vascular endothelial growth factor receptor type 3 (VEGF receptor 3; FIt-4) or neuropilin-1; and is a homo-dimer where two peptide chain units having the amino acid sequence of SEQ. ID. No. 1 are coupled together via an interchain sulfide bond.

In addition, one of vammin protein variants according to the present invention is a VEGF-like protein variant being a variant protein derived from the vammin protein, wherein
the variant protein is a homo-dimer where two peptide chain units receiving modification by replacing an amino acid contained in the amino acid sequence of SEQ. ID. No. 1 with another amino acid are coupled together via an interchain sulfide bond;
said modified amino acid sequence has such a mutation from that of SEQ. ID. No. 1 that the 55^{th} amino acid residue from N-terminus is Arg; and
the variant protein has binding activity to a vascular endothelial growth factor receptor type 2 (VEGF receptor 2; KDR), but does not exhibit any binding affinity to a vascular endothelial growth factor receptor type 1 (VEGF receptor 1; FIt-1), vascular endothelial growth factor receptor type 3 (VEGF receptor 3; FIt-4) or neuropilin-1.

Another one of vammin protein variants according to the present invention is a VEGF-like protein variant being a variant protein derived from the vammin protein, wherein
the variant protein is a homo-dimer where two peptide chain units receiving modification by replacing an amino acid contained in the amino acid sequence of SEQ. ID. No. 1 with another amino acid are coupled together via an interchain sulfide bond;
said modified amino acid sequence has variations from that of SEQ. ID. No. 1 such that the 22^{nd} amino acid residue from N-terminus is Ser, the 55^{th} amino acid residues from N-terminus is Arg, and the 74^{th}, 77^{th} and 79^{th} amino acid residues from N-terminus are any one selected from Arg, Ser or Lys, respectively, with the proviso that a choice resulting in that identical to the amino acid sequence of SEQ. ID. No. 3 is excluded therefrom; and
the variant protein has binding activity to a vascular endothelial growth factor receptor type 2 (VEGF receptor 2; KDR), but does not exhibit any binding affinity to a vascular endothelial growth factor receptor type 1 (VEGF receptor 1; FIt-1), vascular endothelial growth factor receptor type 3 (VEGF receptor 3; FIt-4) or neuropilin-1.

On the other hand, the second embodiment of the novel VEGF-like protein according to the present invention is a vascular endothelial growth factor (VEGF) like protein derived from *Daboia russelli russelli;* VR-1, wherein
the VR-1 protein has binding activity to a vascular endothelial growth factor receptor type 2 (VEGF receptor 2; KDR), but does not exhibit any binding affinity to a vascular endothelial growth factor receptor type 1 (VEGF receptor 1; FIt-1), vascular endothelial growth factor receptor type 3 (VEGF receptor 3; FIt-4) or neuropilin-1; and is a homo-dimer where two peptide chain units having the amino acid sequence of SEQ. ID. No. 2 are coupled together via an interchain sulfide bond.

In addition, one of VR-1 protein variants according to the present invention is a VEGF-like protein variant being a variant protein derived from VR-1 protein; wherein
the variant protein is a homo-dimer where two peptide chain units receiving modification by replacing an amino acid contained in the amino acid sequence of SEQ. ID. No. 2 with another amino acid are coupled together via an interchain sulfide bond;
said modified amino acid sequence has variations from that of SEQ. ID. No. 2 such that the 55^{th} amino acid residues from N-terminus is Arg, and the 74^{th}, 77^{th} and 79^{th} amino acid residues from N-terminus are any one selected from Arg, Ser or Lys, respectively; and
the variant protein has binding activity to a vascular endothelial growth factor receptor type 2 (VEGF receptor 2; KDR), but does not exhibit any binding affinity to a vascular endothelial growth factor receptor type 1 (VEGF receptor 1; FIt-1), vascular endothelial growth factor receptor type 3 (VEGF receptor 3; FIt-4) or neuropilin-1.

Furthermore, the present invention also provides a process for preparing vammin or VR-1 protein.

Hence, a process for preparing vammin protein according to the present invention is a process for purifying and isolating the vammin protein from the venom of *Vipera ammodytes ammodytes,* comprising the step of purifying vammin protein included in said venom collected from the snake by means of multi-step chromatography; and
wherein the vammin protein thus isolated shows a band at 25.8 kDa in SDS-PAGE analysis under non-reductive conditions.

On the other hand, a process for preparing VR-1 protein according to the present invention is a process for purifying and isolating the VR-1 protein from the venom of *Daboia russelli russelli,* comprising the step of purifying VR-1 protein contained in said venom collected from the snake by means of multi-step chromatography; and
wherein VR-1 protein thus isolated shows a band at 25.2 kDa in SDS-PAGE analysis under non-reductive conditions.

Additionally, the present invention also provides inventions of use for the VEGF-like proteins according to the present invention in the field of medicine.

For instance, an embodiment of the inventions for use of the VEGF-like protein according to the present invention is use of the venom-derived VEGF-like proteins or variant proteins thereof according to the present invention as defined above or HF protein having specific binding activity to KDR which is a homo-dimer composed of two peptide chains having the amino acid sequence of SEQ. ID. No. 3, as an active ingredient possessing hypotensive effect for preparing a pharmaceutical composition comprising a hypotensive agent,
wherein said pharmaceutical composition comprises, as the active ingredient possessing hypotensive effect, at least one VEGF-like protein selected from the group consisting of said HF protein, the vammin protein, the vammin protein variant, the VR-1 protein and the VR-1 protein variant mentioned above in combination with a carrier therefor.

Another embodiment of the inventions for use of the VEGF-like protein according to the present invention is use of the venom-derived VEGF-like proteins or variant proteins thereof according to the present invention as defined above or HF protein having specific binding activity to KDR which is a homo-dimer composed of two peptide chains having the amino acid sequence of SEQ. ID. No. 3, as the VEGF-like protein ingredient for preparing a pharmaceutical composition usable for the purpose of treating hepatitis comprising an active ingredient exhibiting effect for inducing KDR-mediated protective action on hepatic cells which utilizes effect of cell proliferation caused by binding of the VEGF-like protein to KDR,
wherein said pharmaceutical composition comprises, as the active ingredient exhibiting effect for inducing KDR-mediated protective action on hepatic cells, at least one VEGF-like protein selected from the group consisting of said HF protein, the vammin protein, the vammin protein variant, the VR-1 protein and the VR-1 protein variant mentioned above in combination with a carrier therefor.

Further embodiment of the inventions for use of the VEGF-like protein according to the present invention is use of the venom-derived VEGF-like proteins or variant proteins thereof according to the present invention as defined above or HF protein having specific binding activity to KDR which is a homo-dimer composed of two peptide chains having the amino acid sequence of SEQ. ID. No. 3, as the VEGF-like protein ingredient for preparing a pharmaceutical composition usable for the purpose of treating vascular endothelial damage associated with percutaneous transluminal coronary intervention (PCI) being one of surgical treatments to arteriosclerosis or of treating an ischemic disease, which composition utilizes vasorelaxation effect, inhibition of platelet aggregation, antithrombotic effect and antiinflammatory effect stimulated by KDR-mediated activation of NO synthetase caused by binding of the VEGF-like protein to KDR,
wherein the pharmaceutical composition comprises, as the VEGF-like protein ingredient possessing binding activity to KDR, at least one VEGF-like protein selected from the group consisting of said HF protein, the vammin protein, the vammin protein variant, the VR-1 protein and the VR-1 protein variant mentioned above in combination with a carrier therefor.

Furthermore, the present invention also provides the invention of use for a gene or nucleic acid molecule having a nucleotide sequence encoding the VEGF-like protein according to the present invention in the field of medicine.

Specifically, an embodiment of the inventions of use for the gene or nucleic acid molecule having a nucleotide sequence encoding the VEGF-like protein or variant protein thereof according to the present invention is use of a gene or nucleic acid molecule having a nucleotide sequence encoding the venom-derived VEGF-like protein or variant protein thereof according to the present invention as defined above or HF protein having specific binding activity to KDR which is a homo-dimer composed of two peptide chains having the amino acid sequence of SEQ. ID. No. 3, as the gene or nucleic acid molecule having a nucleotide sequence encoding the VEGF-like protein, which is contained in the form of a vector for gene recombination where the gene is inserted into a recombinable vector, for recombinational expression of the VEGF-like protein in a host cell from the gene or nucleic acid molecule having a nucleotide sequence encoding a VEGF-like protein having binding activity to KDR,
wherein, as said gene or nucleic acid molecule having a nucleotide sequence encoding the VEGF-like protein, a gene or nucleic acid molecule having a nucleotide sequence encoding at least one VEGF-like protein selected from the group consisting of said HF protein, the vammin protein, the vammin protein variant, the VR-1 protein and the VR-1 protein variant mentioned above is contained in the form of a gene recombination vector where the gene is inserted into the recombinable vector to perform recombinant expression of the VEGF-like protein in the host cell.

On the other hand, the present invention also provides the invention of use for the VEGF-like protein according to the present invention in the biochemical field.

Specifically, another embodiment of the invention of use for a VEGF-like protein according to the present invention is use of the venom-derived VEGF-like proteins or variant proteins thereof according to the present invention as defined above or HF protein having specific binding activity to KDR which is a homo-dimer composed of two peptide chains and having the amino acid sequence of SEQ. ID. No. 3, as a VEGF-like protein reagent having binding affinity to the KDR, for the construction of an *in vitro* assay system for inhibitory effect on binding to KDR to screen a compound having inhibitory effect on binding to KDR of the VEGF-like protein possessing binding affinity to KDR,
wherein the *in vitro* assay system for inhibitory effect on binding to KDR is an assay system evaluating blocking activity of a test compound present in the system on the binding by detecting change in the amount bound to KDR as to the VEGF-like protein reagent possessing binding affinity to KDR, and
wherein at least one of the VEGF-like proteins selected from the group consisting of said HF protein, the vammin protein, the vammin protein variant, the VR-1 protein and the VR-1 protein variant mentioned above is used as the VEGF-like protein reagent possessing binding affinity to KDR.

In addition to the use of said HF protein or a VEGF-like protein or variant protein thereof according to the present invention in the construction of said assay system for inhibitory effect on binding, the present invention also provides the invention. of use in which said HF protein or the VEGF-like protein or variant protein thereof according to the present invention are employed in preparing a pharmaceutical composition with use of a compound having inhibitory effect on binding affinity to KDR of the VEGF-like protein according to the present invention, which compound is selected by utilizing the assay system for binding inhibitory effect.

Specifically, an embodiment of the method for using a VEGF-like protein or variant protein thereof according to the present invention is use of the venom-derived VEGF-like proteins or variant proteins thereof according to the present invention as defined above or HF protein having specific binding activity to KDR which is a homo-dimer composed of two peptide chains and having the amino acid sequence of SEQ. ID. No. 3 in preparing a pharmaceutical composition comprising a compound having inhibitory effect on binding affinity to KDR of the VEGF-like protein as an active ingredient for treating an angiogenesis disease,
wherein said pharmaceutical composition comprises, as an active ingredient, a compound having blocking activity on the binding affinity to KDR selected by utilizing the aforementioned assay system for inhibitory effect on binding to KDR according to the present invention, in the mechanism for the inhibition of said binding to KDR, which compound is bound on the site for ligand-binding in KDR to block the ligand-binding, or is bound on the site other than the site for ligand-binding in KDR to change the conformation of the binding site, resulting in the prevention of ligand-binding, and
wherein in the selection by using the assay system for inhibitory effect on binding to KDR, at least one of the VEGF-like proteins selected from the group consisting of said HF protein, the vammin protein, the vammin protein variant, the VR-1 protein and the VR-1 protein variant mentioned above is used as the VEGF-like protein reagent possessing binding affinity to KDR.

In connection with the use of the venom-derived VEGF-like proteins or variant proteins thereof according to the present invention as defined above or HF protein having specific binding activity to KDR which is a homo-dimer composed of two peptide chains and having the amino acid sequence of SEQ. ID. No. 3 in preparing the pharmaceutical composition comprising a compound having inhibitory effect on binding affinity to KDR of the VEGF-like protein as an active ingredient for treating an angiogenesis disease, the pharmaceutical composition is thus executed to be applicable to drug therapy for angiogenesis diseases with various symptoms such as solid tumor growth, diabetic retinopathy, premature infant retinitis, rheumatoid arthritis and psoriasis.

The novel VEGF-like proteins according to the present invention: vammin and VR-1 do not bind to FIt-1 (VEGF receptor 1) at all, but bind to KDR(VEGF receptor 2) with higher affinity. Thus, they can be employed, in place of a natural VEGF-A protein, as drug with use of its potent nitrogen monoxide (NO)-dependent hypotensive effect and/or angiogenesis promoting effect induced by binding of the VEGF-like protein to KDR, for instance, a hypotensive agent, therapeutic drug for an ischemic disease utilizing the platelet aggregation inhibitory and anti-thrombogenic activity; a therapeutic drug for vascular endothelial cell damage which is applicable to such treatment of a vascular inner wall damaged by percutaneous transluminal coronary intervention (PCl) that is typical one of surgical procedures for arteriosclerosis; or a therapeutic drug for hepatitis in which hepatic cells are to be protected utilizing the anti-inflammatory effect. In these applications, their use may be capable of reducing significantly side effects such as excessive proliferation of hepatic cells or hypertrophy of the liver that is caused by ligand-binding to FIt-1, which may be concerns during long term administration. In comparison with a VEGF-E protein and mutated proteins form VEGF-A, which all have no binding affinity to FIt-1, but specifically bind to KDR, vammin and VR-1 are superior in hypotensive effect.

### Brief Description of Drawings

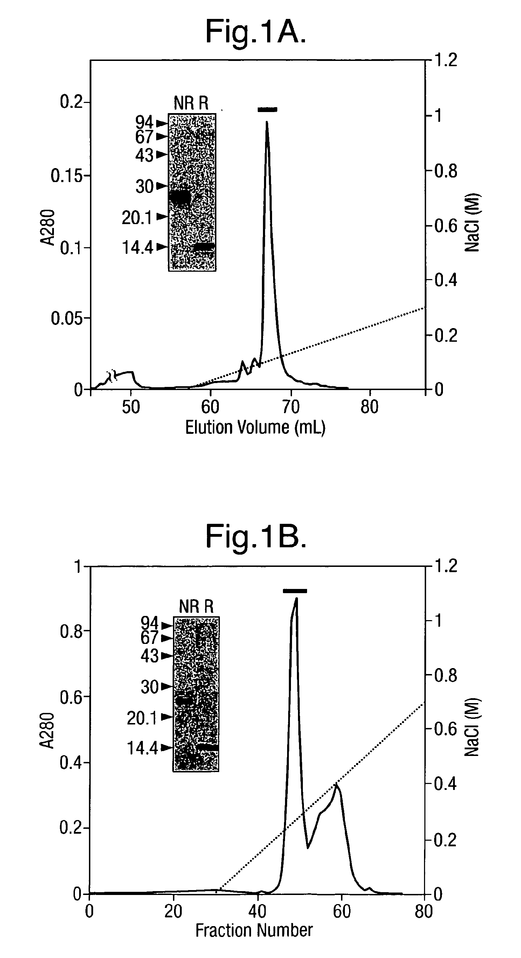

FIG. 1-A shows elution fractions containing vammin (indicated by a bar in the chart) in Mono S column chromatography used for Step 5 of the process for purifying vammin explained in an example as described below with SDS-PAGE analysis result thereof, and FIG. 1 B shows elution fractions containing VR-1 (indicated by a bar in the chart) in SP-Sepharose High Performance column chromatography used for Step 4 of the process for purifying VR-1 as in an example described below with SDS-PAGE analysis result thereof. In the SDS-PAGE results inserted, lanes indicate the followings; NR: electrophoresis results under non-reductive conditions and R: electrophoresis results under reductive conditions, respectively.

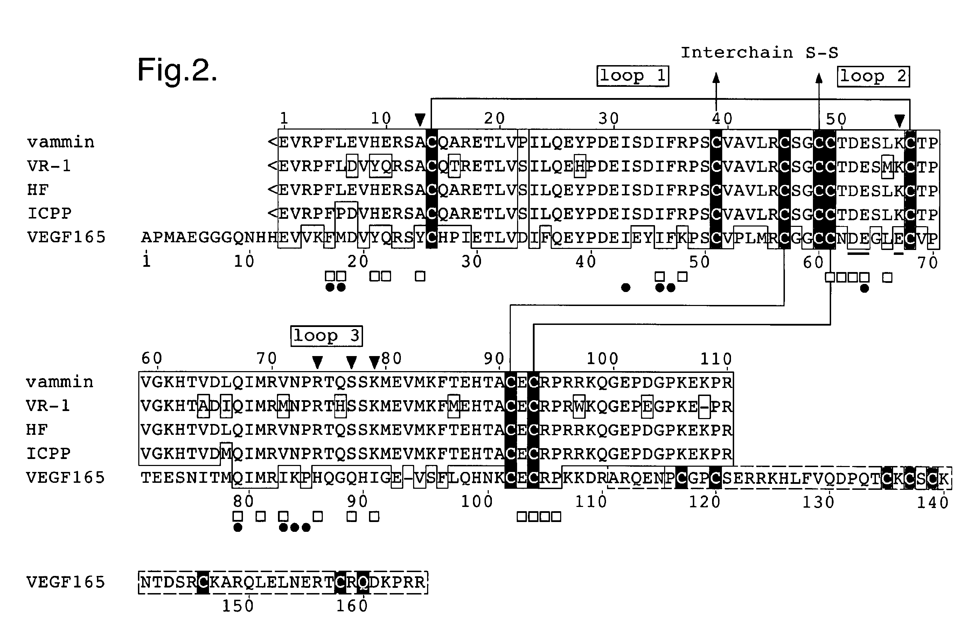

FIG. 2 shows resulted alignment for the amino acid sequences of peptide chains of the venom-derived VEGF-like proteins and human VEGF165. The amino acid residues being identical within the venom-derived VEGF-like proteins are indicated by shadowing; cysteine residues conserved are indicated by reversed characters, intra-chain disulfide bonds and inter-chain disulfide bonds are also illustrated; and portions of loops in the peptide chain of VEGF165 forming the cystine-knot motif are designated with oblong boxes. A heparin binding site in VEGF165 is enclosed by a dotted line. The essential residues for binding to KGR receptor in human VEGF165 are indicated by **•;** the essential residues for binding to FIt-1 are indicated by □; and the sites for amino-acid replacement contributing to reduction in binding affinity to FIt-1 in a venom-derived VEGF-like protein are indicated by ▼ .

Other notations mean as follows:

HF: a venom-derived hypotensive factor of *Vipera aspis aspis;* ICPP: a venom-derived increasing capillary permeability protein of *Vipera lebetina;* and VEGF165: human vascular endothelial growth factor 165 (GenBank accession number: AAM03108).

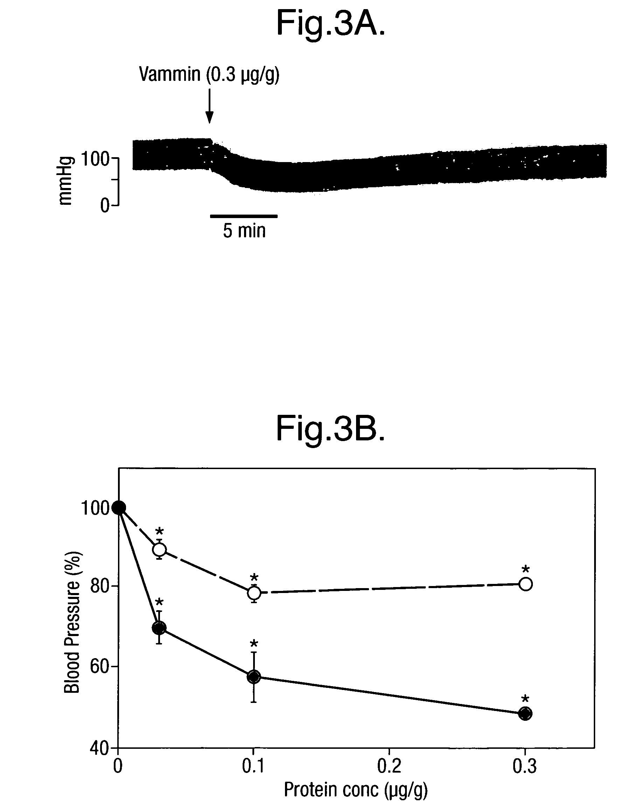

FIG. 3(A) is a recording chart over time for a rat arterial blood pressure after intravenous injection of vammin in a dose of 0.3 µg/g, and FIG. 3(B) is a plot showing dosage dependency of lowering rates of a systolic blood pressure (SBP; white dot) and a diastolic blood pressure (DBP; black dot) in a rat arterial blood pressure post to intravenous injection of vammin. The data shown in FIG. 3(B) are expressed as an average ± standard deviation calculated from the results obtained for three or five animals per group. *: P<0.05 in a t-test for significance in comparison with a control (negative control group).

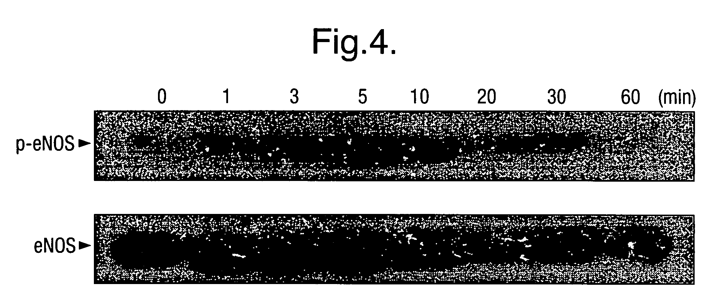

Fig. 4 shows the time-course of the activation of No synthetase by phosphorylation. Serum-free bovine coronary artery endothelial cells (CAECs) were treated with vammin (1 nM). After a given time passed away, the cells were washed quickly with ice-cooled PBS twice and lysed in a lysis buffer. Each cell lysate was tested for the presence of phosphorylated eNOS (on Ser¹¹⁷⁷, shown in upper of Fig. 4) and the total eNOS (shown in lower of Fig. 4) by immunoblotting using antibodies thereto.

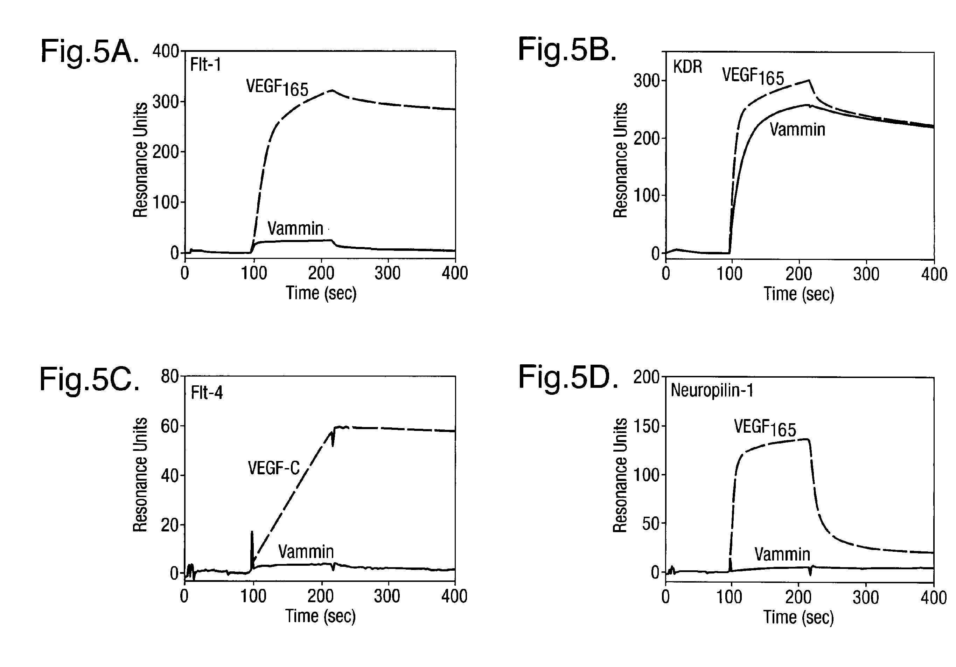

FIG. 5a shows comparison between the changes in resonance signals measured in the processes of association to and dissociation from a receptor molecule: Flt-1-IgG immobilized on a CM5 sensor chip for human VEGF165 (10 nM; dotted line) and vammin (10 nM; straight line). FIG. 5b shows comparison between the changes in resonance signals measured in the processes of association to and dissociation from a receptor molecule: KDR-IgG immobilized on a CM5 sensor chip for human VEGF165 (30 nM; dotted line) and vammin (30 nM; straight line). FIG. 5c shows comparison between the changes in resonance signals measured in the processes of association to and dissociation from a receptor molecule: FIt-4 (VEGF receptor type 3) immobilized on a CM5 sensor chip for VEGF-C156S variant (500 nM; dotted line) and vammin (500 nM; straight line). FIG. 5d shows comparison between the changes in resonance signals measured in the processes of association to and dissociation from a receptor molecule: neuropilin-1-IgG immobilized on a CM5 sensor chip for human VEGF165 (30 nM; dotted line) and vammin (30 nM; straight line).

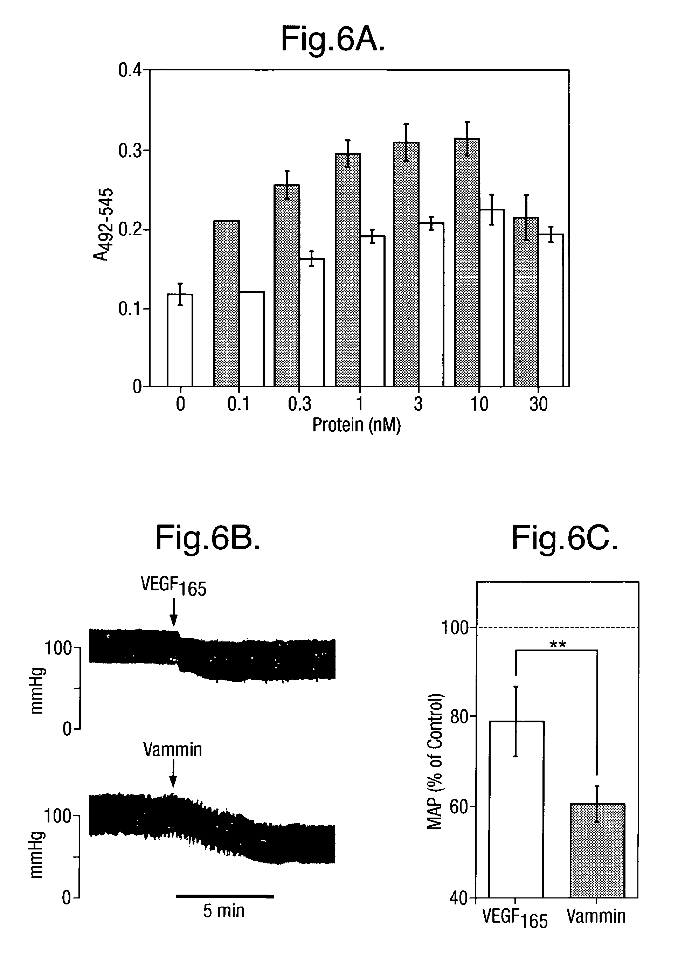

FIG. 6 shows the results of comparison between a venom-derived VEGF-like protein vammin and human VEGF165 for physiological effects. FIG. 6a shows comparison between stimulating effects on proliferation of vascular endothelial cells of vammin and human VEGF165. Each concentration of vammin or VEGF165 was added to bovine aortic endothelial cells cultured in 0.1 % fetal calf serum (3,000 cells/well). After six days, the numbers of the alive cells were counted by WST-8 method. Gray column: vammin, and white column: VEGF165. FIGs. 6b and 6c compare hypotensive effect of vammin and VEGF165 on a rat carotid arterial pressure. FIG. 6b compares the time-course changes in a blood pressure that were invasively detected for in the cases of administration of vammin (0.1 µg/g) or VEGF165 (0.1 µg/g) from a rat femoral vein. FIG. 6c compares the observed maximum reductions in an average arterial pressure (MAP) post to the intravenous administration of vammin (0.1 µg/g) or VEGF165 (0.1 µg/g). **P<0.05, n=3 to 4.

### DETAILED DESCRIPTION OF PREFERRED EMBODIMENTS OF THE PRESENT INVENTION

This invention will be explained in detail hereafter.

The present invention provides a VEGF-like protein purified and isolated from a venom of *Vipera ammodytes ammodytes:* vammin and a VEGF-like protein purified and isolated from a venom of *Daboia russelli russelli:* VR-1, which are revealed, from the analytical results described later in Examples, to be a homo-dimer being composed of two 110 amino-acid peptide chains coupled via an interchain disulfide bond, and a homo-dimer being composed two 109 amino-acid peptide chains coupled via an interchain disulfide bond, respectively. Specifically, the primary structure (amino acid sequence) of the 110 amino-acid peptide chain constituting vammin is presented in SEQ. ID. No. 1.

SEQ. ID. No. 1: the primary structure of the 110 amino-acid
peptide chain of vammin:
EVRPFLEVHE RSACQARETL VPILQEYPDE
ISDIFRPSCV AVLRCSGCCT DESLKCTPVG
KHTVDLQIMR VNPRTQSSKM EVMKFTEHTA
CECRPRRKQG EPDGPKEKPR.

The primary structure (amino acid sequence) of the 109 amino-acid peptide chain constituting VR-1 is presented in SEQ. ID. No. 2.

SEQ. ID. No. 2: the primary structure of the 109 amino-acid
peptide chain of VR-1:
EVRPFLDVYQ RSACQTRETL VSILQEHPDE
ISDIFRPSCV AVLRCSGCCT DESMKCTPVG
KHTADIQIMR MNPRTHSSKM EVMKFMEHTA
CECRPRWKQG EPEGPKEPR.

As described below in Examples, our amino acid analysis has demonstrated that the primary structure of the 110 amino-acid peptide chain in the VEGF-like protein: vammin purified and isolated from the venom of *Vipera ammodytes ammodytes* exhibits quite higher homology to the primary structure of HF isolated from a venom of *Vipera aspis aspis* presented in SEQ. ID. No. 3 or the primary structure of ICPP isolated from a venom of *Vipera lebetina.* presented in SEQ. ID. No. 4, both of which are homo-dimers composed of two 110 amino-acid peptide chains coupled with an interchain disulfide bond:

SEQ. ID. No. 3: the primary structure of the 110 amino-acid
peptide chain of HF
EVRPFLEVHE RSACQARETL VSILQEYPDE
ISDIFRPSCV AVLRCSGCCT DESLKCTPVG
KHTVDLQIMR VNPRTQSSKM EVMKFTEHTA
CECRPRRKQG EPDGPKEKPR,

SEQ. ID. No. 4: the primary structure of the 110 amino-acid
peptide chain of ICPP
EVRPFPDVHE RSACQARETL VSILQEYPDE
ISDIFRPSCV AVLRCSGCCT DESLKCTPVG
KHTVDMQIMR VNPRTQSSKM EVMKFTEHTA
CECRPRRKQG EPDGPKEKPR.

Furthermore, after fully investigating correlation between physiological effects of the series of venom-derived VEGF-like proteins and their amino acid sequences, it has been found that, among the coincident portion of these amino acid sequences, one feature in an amino acid sequence significantly contributing to the common property that they do not bind to FIt-1 while retaining higher affinity to KDR is that they retain 5 amino acids of Ala¹³, Lys⁵⁵, Arg⁷⁴, Ser⁷⁷ and Lys⁷⁹ in SEQ. ID. No. 1.

Furthermore, it may be concluded that as for an artificial variant of vammin protein having physiological effect substantially comparable to a natural vammin, the primary structure of a peptide chain such as that SEQ. ID. No. 5 is preferably used in production of the homo-dimer composed of two peptide chains coupled via an interchain disulfide bond: 
SEQ. ID. No. 5: the primary structure of a 110 amino-acid peptide
chain of a vammin variant having a mutation of Lys55→Arg
EVRPFLEVHE RSACQARETL VPILQEYPDE
ISDIFRPSCV AVLRCSGCCT DESLRCTPVG
KHTVDLQIMR VNPRTQSSKM EVMKFTEHTA
CECRPRRKQG EPDGPKEKPR;
 and that as for an artificial variant of VR-1 protein having physiological effect substantially comparable to a natural VR-1, the primary structure of a peptide chain such as those of SEQ. ID. No. 6 and SEQ ID. No. 7 or primary structures containing combination of these single amino acid replacements are preferably used in production of the homo-dimer composed of two peptide chains coupled via an interchain disulfide bond:

SEQ. ID. No. 6: the primary structure of a 109 amino-acid peptide
chain of a VR-1 variant having a mutation of Lys55→Arg
EVRPFLDVYQ RSACQTRETL VSILQEHPDE
ISDIFRPSCV AVLRCSGCCT DESMRCTPVG
KHTADIQIMR MNPRTHSSKM EVMKFMEHTA

CECRPRWKQG EPEGPKEPR;

SEQ. ID. No. 7: the primary structure of a 109 amino-acid peptide
chain of a VR-1 variant having a mutation of Ser22→Pro
EVRPFLDVYQ RSACQTRETL VPILQEHPDE
ISDIFRPSCV AVLRCSGCCT DESMKCTPVG
KHTADIQIMR MNPRTHSSKM EVMKFMEHTA
CECRPRWKQG EPEGPKEPR.

As to means for purifying and isolating Vammin and VR-1 proteins according to the present invention from their natural sources: snake venpm, which is capable of retaining the form of homo-dimer composed of two peptide chains being coupled via an interchain disulfide bond, it is preferable to utilize such purification process by means of multistep column chromatography under the conditions without a reducing agent. For example, the purification process with use of the multistep column chromatography is preferably conducted in such manner as described in Example 1 wherein the process comprises:
Step 1: Superdex 200pg gel filtration chromatography;
Step 2: HiTrap heparin High Performance column chromatography;
Step 3: Q-Sepharose High Performance column chromatography;
Step 4: SP-Sepharose High Performance column chromatography; and
Step 5: Mono S column chromatography,
whereby a higher purity can be easily and efficiently achieved. Besides, as long as principle for the removal and separation of impurities of each step is substantially same to those used in the process described above, parameters such as a specific step sequence, the types of columns used, or a buffer and elution condition used for each column purification can be appropriately modified.

On the other hand, HF protein and vammin and VR-1 proteins according to the present invention as well as artificial variant proteins thereof are produced by gene recombination. Specifically, in Examples below, an expression system used for gene recombination such as a recombinant human VEGF165 is used also for recombining nucleic acid molecules encoding the peptide chains of the vammin protein, the VR-1 protein and artificial variant proteins thereof into the expression system, which allows a recombinant protein to be obtained in the complete form where structure of a homo-dimer is composed of two peptide chains being coupled via an interchain disulfide bond and a loop structure in each chain of the peptide chains is formed due to the conserved cystein knot motif as in the wild VEGF protein. In addition, the purification/isolation therein is appropriately selected, depending on the expression system and host used. In the case of these VEGF-like proteins that require, for example, the folding process where after translation into the corresponding peptide chains, coupling via appropriate intrachain and interchain disulfide bonds is made to form the homo-dimer, in this view, in the recombinant production thereof, it is desirable to use an eukaryocyte such as yeast, fungi, plant cells, plants, animal cells and insect cells having, in the folding and assembly machinery or secretion machinery for a mature protein consisting of multiple peptide units, a processing mechanism for such as coupling via appropriate intrachain and interchain disulfide bonds post to the translation into the corresponding peptide chains mentioned above, as a host used in the expression system.

Furthermore, for demonstrating that as for the recombinant vammin protein, the VR-1 protein and artificial variant proteins thereof, the amino acid sequence obtained is indeed identical to that desired, the analytic procedure for an amino acid sequence described in Examples below is available. In such a case, the procedure may be modified; for example, these steps of dissociating a homo-dimer into individual peptide chains, alkylation of a cysteine -SH contained therein and fragmentation of the peptide are carried out by the methods as described in Examples, and then an alternative technique may be used as a method of analyzing the amino acid sequences of the fragments.

On the other hand, with respect to a nucleic acid molecule encoding a peptide chain of the HF protein, the vammin protein, the VR-1 protein or artificial protein thereof used in the above recombinational production, its full nucleotide sequence can be prepared by chemical synthesis according to the desired amino acid sequence therefor and also taking account of, for instance, codon usage in a host for the recombinational expression. Alternatively, such a molecule may be obtained as an mRNA or cDNA collected from its source, a snake, or a desired DNA may be collected from a genome gene utilizing a corresponding probe or PCR primer. The codon usage in the encoding nucleic acid molecule collected from the source snake may be different from that in a host for recombinational expression. In such a case, the codons therein may be desirably subjected to optimization or replacement in accordance with a conventional technique. On the other hand, a nucleic acid molecule encoding a peptide chain of an artificial variant protein may be appropriately produced by site-specific mutation.

Furthermore, . the nucleic acid molecule encoding a peptide chain of the HF protein, the vammin protein, the VR-1 protein or artificial variant protein thereof may be applicable to use of so-called gene therapy where the molecule is introduced in a human body for allowing human cells to secrete the VEGF-like protein. For this purpose, techniques using vector systems developed for a variety of gene therapeutical applications may be used as a vector used for gene recombination, which is genetically introduced in a human cell for secretion of product of said gene. For example, a retrovirus type vector system, which is developed for gene therapy for a variety of diseases caused by a genetic defect, may be used.

On the other hand, the HF protein, vammin and VR-1 proteins according to the present invention as well as artificial variant proteins thereof may be applied as a hypotensive agent by directly introducing a solution containing the VEGF-like protein into a body via such path as intravenous injection, utilizing their excellent hypotensive effect. There has been reported that a KDR-specific human VEGF variant induces protection of hepatocytes mediated by KDR (LeCouter, J. et al., Science, 299, 890-893 (2003)). Thus, the vammin and the VR-1 proteins as well as artificial variant proteins thereof may be also applied to treatment of hepatitis utilizing their similar protective effect on hepatocytes mediated by KDR.

Furthermore, the HF protein and vammin and VR-1 proteins according to the present invention as well as artificial variant proteins thereof may be applied to treating a blood vessel damaged by percutaneous transluminal coronary intervention (PCI), which is a therapy for arteriosclerosis, by introducing the protein into the body via such path as intravenous injection, utilizing their function as a growth stimulating factor for vascular endothelial cells. In addition, they may be applied to treating an ischemic disease, utilizing the vasorelaxation effect induced by KDR-mediated activation of NO synthetase, inhibition of platelet aggregation, antithrombotic effect and antiinflammatory effect, which effects are caused by binding of the VEGF-like protein to KDR.

In these uses, the HF protein and the vammin and VR-1 proteins according to the present invention as well as artificial variant proteins thereof are suitably administered in such manner that the proteins are administered directly into blood flow to be delivered to the site of action. Generally, a pharmaceutical composition therefor is preferably prepared in a dosage form adapted to intravenous administration. Specifically, the composition may be formulated in a dosage form generally used for intravenous administration of proteins having various physiological effects; for example, an intravenous injectable solution or an infusion solution. A unit dose thereof is determined as appropriate, depending on a treatment application. It is preferable to set up the dose such that the dose provides its sufficient level in blood to achieve desired physiological effect, based on the estimated total amount of blood in the subject (patient) and taking some factors such as the status, severity of the symptom, sex, age, body weight and other health parameters of the patient into consideration. When using the HF protein and the vammin and VR-1 proteins according to the present invention as well as artificial variant proteins thereof in a dosage form of an intravenous injection solution, a dose is generally chosen in the range of 0.03 to 0.3 mg/kg body weight, preferably in the range of 0.1 to 0.2 mg/kg body weight.

In recombinant production of the HF protein and the vammin and VR-1 proteins according to the present invention as well as artificial variant proteins thereof, it is necessary to quantitatively evaluate the physiological effects of the recombinant proteins. In such evaluation step, the evaluation procedure described in Examples, such as a method for determining hypotensive capability, an analytical method for binding affinity to KDR or FIt-1 or an immunoassay analysis for getting sight of activation of NO synthetase, may be preferably used. Specific procedures and conditions in such an evaluation may be appropriately modified as long as quantitative evaluation can be obtained similarly. It may be also possible to employ another assay system or evaluation procedure which allows for equivalent quantitative evaluation of physiological effect to the above evaluation process.

When applying the vammin and VR-1 proteins or artificial variant proteins thereof according to the present invention as well as the HF protein to treatment of an ischemic disease utilizing their vasorelaxation effect induced by KDR-mediated activation of NO synthetase, inhibition of platelet aggregation, antithrombotic effect and antiinflammatory effect caused by binding of the VEGF-like protein to KDR, there may be cases requiring continuous administration. In such a case, they can be applied to treatment of an ischemic disease in the form of gene therapy where a gene encoding any of the VEGF-like proteins is artificially synthesized and the gene is introduced into a somatic cell of a subject (patient) by means of a vector to in vivo produce the proteins in the cell.

In addition, as the vammin and VR-1 proteins or artificial variant proteins thereof according to the present invention as well as the HF protein have specific feature in binding to KDR or Flt-1, they can be used as a standard reagent for analysis of binding affinity to KDR or Flt-1 in an evaluation process or system for binding affinity to the receptors by means of surface plasmon resonance described in Example 1 below. On the contrary, it is possible to screen a binding inhibitor exhibiting inhibitory effect to the binding on KDR of the HF protein, the vammin or VR-1 protein or artificial variant protein thereof according to the present invention, by utilizing such an evaluation process or system for binding affinity to receptor by means of surface plasmon resonance. Particularly, for example, in terms of an inhibition mechanism for such a binding inhibitor, known parameters for binding properties of the vammin or VR-1 protein or artificial variant protein thereof according to the present invention as well as the HF protein may be used for analysis, to determine whether the mechanism involves its action on the VEGF-like protein which affects its binding properties or its action on KDR which has influences on binding of the VEGF-like protein thereto. Furthermore, inhibition property parameters of a binding inhibitor screened may be estimated on the basis of the measurement results under various conditions.

In addition, after confirming that a binding inhibitor exhibiting effect blocking the binding to KDR of the HF protein or the VEGF-like protein according to the present invention also has inhibitory effect on binding to KDR of an endogenous VEGF protein such as human VEGF through the same inhibition mechanism, the inhibitor may be used as an angiogenesis inhibitor applicable to treatment of, for example, an angiogenesis disease, such as solid tumor growth, diabetic retinopathy, premature infant retinitis, chronic rheumatoid arthritis or psoriasis, which is caused by binding to KDR of the endogenous VEGF in a human body. For example, this type of angiogenesis inhibitors may be searched by routinely repeated experiments where compounds in a known compound library are subjected to random screening by applying the above screening procedure to determine whether they have desired inhibiting effect. The compound library to be screened may be that of synthetic compounds or compounds isolated from the natural sources, and that of low-molecular weight compounds or high-molecular weight compounds. In terms of the inhibition mechanism for said binding inhibitor, there are assumed such mechanisms where the compound may be bound on the site for ligand-binding in KDR to block the ligand-binding, or may be bound on the site other than the site for ligand-binding to change the conformation of the binding site, resulting in the prevention of ligand-binding. Compounds having any of these inhibition mechanisms may be usable for the aim.

After confirming that the angiogenesis inhibitor searched causes no reluctant side effects when being administered to a subject (patient), it is preferably used in an administration route and a dosage form appropriately selected depending on a site or symptom of a targeted angiogenesis disease, such as solid tumor growth, diabetic retinopathy, premature infant retinitis, chronic rheumatoid arthritis and psoriasis.

### EXAMPLES

The present invention will be more specifically explained with reference to Examples. The particular examples presented below are included in examples of the best mode of the present invention, but the technical scope of the present invention is not limited to these specific embodiments in any manner.

In the examples, there will be more specific description in terms of:
a procedure for purifying and isolating the VEGF-like protein; vammin and VR-1 from the individual snake venoms;
demonstrating that the vammin and the VR-1 are homo-dimers composed of two peptide chains being coupled via a disulfide bond and determining the amino acid sequences of the peptide chains;
binding properties thereof to the VEGF receptor and the features on the amino acid sequence involved in the binding properties; and
hypotensive effect of the venom-derived VEGF-like protein; vammin, VR-1 and HF and elucidation of the mechanism of action.

### 1. Preparation process for an anti-HF antibody

In the process for purification and isolation of a VEGF-like protein; vammin and VR-1 from the snake venoms described below, an anti-HF antibody having cross-reactivity is used in ELISA and Western blotting analyses for these venom-derived VEGF-like proteins, which methods are employed as means for evaluating the purity obtained at each stage of the purification steps.

The anti-HF antibody was prepared from a rabbit subjected to immune injection of HF isolated from a venom of *Vipera aspis aspis.* First, a complete adjuvant and 180 µg of purified HF were injected to a male New Zealand white rabbit to immunize therewith. After followed by twice of booster-immunizations with 14 days interval, whole the blood was gathered from the animal. After separating the serum by centrifugation, an antibody titer for the anti-HF IgG antibody contained therein was measured by ELISA. Finally, an anti-serum of which the 10,000-fold dilution was still reactive to the antigen HF was obtained.

### 2. Purification process for the venom-derived VEGF-like proteins .

Purification of the VEGF-like protein contained in each snake venom was carried out at 4°C using a chromatograph: FPLC system equipped with a device for detection: AKTA explorer 10S (Amersham Biosciences). A protein concentration in an elution fraction from each chromatography column was measured by absorbance detected at 280 nm.

Purification of the VEGF-like protein; vammin from venom of *Vipera ammodytes ammodytes* was attained through the following five-step chromatography process.

First, 200 mg of the crude venom of *Vipera ammodytes ammodytes* was dissolved in 2 mL of 50 mM Tris-HCl pH 8.0.

### Step 1: Superdex 200pg gel filtration chromatography

The crude snake venom solution was applied at a flow rate of 2 mL/min to a Superdex 200pg gel filtration chromatography column (2.6 cmφ x 60 cm L) being equilibrated with a 50 mM Tris-HCl pH 8.0 buffer in advance. Detection and concentration measurment of vammin contained in each elution fraction was conducted by ELISA using the anti-HF antibody. Fractions containing vammin were pooled to deliver to the next purification step.

### Step 2: HiTrap heparin High Performance column chromatography

The vammin fractions pooled at the previous step were applied to a HiTrap heparin High Performance column being equilibrated with the Tris-HCl buffer. The column was eluted with a 20-column volume eluent under the linear gradient condition up to 0.6 M NaCl at a flow rate of 1 mL/min. ELISA analysis of each elution fraction indicated that vammin was eluted around 0.3 M NaCl. The pooled vammin-containing fractions were desalted by dialysis with 50 mM Tris-HCl pH 8.0. The dialyzed solution was subjected to the next purification step.

### Step 3: Q-Sepharose High Performance column chromatography

The dialyzed solution prepared at the previous step was applied to a Q-Sepharose High Performance column (1.6 cmφ x 10 cm L). ELISA analysis indicated that vammin was contained in the passing-through fractions. The pooled passing-through fractions were dialyzed with 20 mM imidazole-HCl pH 6.0. After buffer replacement by the dialysis, the dialyzed vammin-containing solution was used in the next purification step.

### Step 4: SP-Sepharose High Performance column chromatography

The dialyzed vammin-containing solution obtained in the previous step was applied to a SP-Sepharose High Performance column (1.6 cmφ x 11 cm L) equilibrated with said imidazole-HCl buffer. The column was eluted with a 4-column volume eluent under the linear gradient condition up to 0.3 M NaCl at a flow rate of 1 mL/min.

ELISA analysis of each elution fraction indicated that vammin was eluted around 0.1 M NaCl. The vammin-containing fractions were pooled.

### Step 5: Mono S column chromatography

Finally, the pooled vammin-containing fraction solution obtained in the previous step was purified with a Mono S column to give a purified standard sample.

From 200 mg of the crude snake venom, 1.3 mg of the purified vammin protein was obtained.

In addition, purification of the VEGF-like protein; VR-1 from a venom of *Daboia russelli russelli* was carried out by using the procedure and conditions similar to those of said purification process for vammin, whereby 8.2 mg of the purified VR-1 protein was obtained from 1900 mg of the crude snake venom.

Using a standard solution of the protein purified, the protein concentration was determined by amino acid analysis and bicinchoninic acid protein assay (Pierce).

FIG. 1 (A) shows SDS-PAGE analysis results for elution fractions containing vammin (fractions labeled with a bar in the chart) in Mono S column chromatography of Step 5 in the process for purifying vammin mentioned above. On the SDS-PAGE, vammin gave bands at 14.6-kDa under reductive conditions (R) in the presence of a reducing agent and at 25.8-kDa under non-reductive conditions (NR), suggesting that it is a homo-dimer composed of two peptide chains being coupled via a disulfide bond. FIG. 1(B) shows SDS-PAGE analysis results for elution fractions containing VR-1 (fractions labeled with a bar in the chart) in SP-Sepharose High Performance column chromatography of Step 4 in the process for purifying VR-1. On the SDS-PAGE, VR-1 gave bands at 14.7-kDa under reductive conditions (R) and at 25.2-kDa under non-reductive conditions (NR), also suggesting that it is a homo-dimer composed of two peptide chains being coupled via a disulfide bond.

After reduction, both S-pyridylethylated vammin and VR-1 were eluted as a single peak in reverse phase HPLC. It can be, therefore, concluded that these proteins are homo-dimer proteins which are composed of two peptide chains being coupled via an interchain disulfide bond.

### 3. Amino acid sequencing

5 nmol of VR-1 and vammin were subjected to reductive alkylation by the conventional method (Yamazaki, et al., Biochemistry, 41, 11331-11337 (2002)). The lyophilized S-alkylated peptide chains constituting the homo-dimer proteins were fragmentated by digestion with endoproteases, Lys-C, Asp-N and Arg-C at 37 °C for 24 hours. Each peptide fragment obtained by said enzymic digestion using endoproteases was analyzed by a protein sequencer (Applied Biosystems 473A, 477, Shimadzu PPSQ-21 A). The pyrrolidone ring at N-terminus was opened with methanol-hydrochloric acid treatment (Kawasaki, I. et al., Anal. Biochem., 48, 546-556 (1972)). Separately, a lyophilized S-alkylated peptide chain (250 pmol) was treated with 3N HCl/MeOH at 40 °C for 2 hours, and then the N-terminal amino acid sequence was analyzed by the protein sequencer. Based on the amino acid sequence of each peptide fragment obtained by digestion with these endoproteases and the N-terminal amino acid sequence, the full amino acid sequences of the peptide chains constituting each homo-dimer protein were determined.

The full amino acid sequences determined for vammin and VR-1 are presented in SEQ. ID. Nos. 1 and 2, respectively. Vammin is a homo-dimer protein consisting of two 110-amino acid peptide chains, while VR-1 is a homo-dimer protein consisting of two 109-amino acid peptide chains. FIG. 2 shows the result of alignment of the primary structures of the peptide chains constituting vammin and VR-1 in comparison with the primary structures of the peptide chains constituting the homo-dimer proteins for members of VEGF family (i.e. human VEGF 165, HF, ICPP) reported. In primary structures of the peptide chains constituting the homo-dimer proteins, vammin and VR-1 show homology with the known VEGF family members, and thus cysteine-knot motif is completely conserved therein, which is a characteristic feature of the family of VEGF proteins. Vammin and VR-1 had homologies with human VEGF165 of 47.6 % and 48.1 %, respectively. They had a higher homology with the venom-derived VEGF-like protein (HF, ICPP).

A binding site (Asn⁷⁵) for an N-glycosylation in human VEGF 165 is replaced with another amino acid residue (Thr⁷⁵) in vammin or VR-1 as is for the other venom-derived VEGF-like protein (HF, ICPP) (FIG. 2). All the amino acid residues could be identified by peptide sequencing. It can be thus concluded that the amino acid residue was not modified such as glycosylation or phosphorylation.

There has been found residues essential for binding to a VEGF receptor in human VEGF165 by variant analysis and crystal structure analysis (Keyt, B. A. et al., J. Biol. Chem., 271, 5638-5646 (1996); Muller, Y. A. et al., Proc. Natl. Acad. Sci. USA, 7192-7197(1997); Wiesmann, C. et al., Cell, 91, 695-704 (1997); Fuh, G. et al., J. Biol. Chem., 273, 11197-11204 (1998); Li, B. et al., J. Biol. Chem., 275, 29823-29828 (2000); Pan, B. et al., J. Mol. Biol., 316, 769-787 (2002)). In vammin and VR-1, most of the essential residues for binding to KGR-receptors in human VEGF165 (indicated by •) were also highly conserved, but some of the essential residues for binding to FLt-1 (indicated by □) were replaced by other amino acid residues (FIG. 2). First, Tyr²⁵ in human VEGF165, to which 13^{th} amino acid residue of vammin corresponds, was replaced by Ala. It has been demonstrated that the alanine variant of human VEGF at this site (human VEGF Tyr²⁵→Ala) exhibits about 1/100 binding affinity to FIt-1 without influence on binding to KDR (Muller, Y. A. et al., Proc. Natl. Acad. Sci. USA, 7192-7197 (1997); Pan, B. et al., J. Mol. Biol., 316, 769-787 (2002)). Secondly, the results of three-dimensional structure analysis have demonstrated that three amino acids (His⁸⁶, Gln⁸⁹, Ile⁹¹) in the third loop in human VEGF165, which have been implied to interact with FIt-1 (Wiesmann, C. et al., Cell, 91, 695-704 (1997)), are replaced by Arg⁷⁴, Ser⁷⁷, Lys⁷⁹ in vammin and VR-1. It has been described that alanine mutation of one of the three residues does not affect binding to FIt-1 (Keyt, B. A. et al., J. Biol. Chem., 271, 5638-5646 (1996); Li, B. et al., J. Biol. Chem., 275, 29823-29828 (2000); Pan, B. et al., J. Mol. Biol., 316, 769-787 (2002)). Thirdly, among the negatively charged amino acid residues (Asp⁶³, Glu⁶⁴, Glu⁶⁷; underlined in FIG. 2) in the second loop in VEGF165, which have been implied to be essential for binding to FIt-1 (Keyt, B. A. et al., J. Biol. Chem., 271, 5638-5646 (1996)), one residue (Glu⁶⁷) is replaced by a positively charged residue (Lys⁵⁵) in the venom-derived VEGF-like protein. It can be thus speculated that in VEGF165, replacement of these five residues (Tyr²⁵; Glu⁶⁷; His⁸⁶; Gln⁸⁹; Ile⁹¹) with other amino acids (indicated by ▼ in FIG. 2) may allow the venom-derived VEGF-like protein to be a KDR selective agonist.

### 4. Evaluation of hypotensive activity of the venom-derived VEGF-like proteins; vammin, VR-1 and HF

Hypotensive activity of a venom-derived VEGF-like protein was determined using male Wistar rats (an animal number per group n = 3 or 5, with body weight of 150 to 220 g). After anesthetizing each animal by intraperitoneal injection of ethyl carbamate (1 g/kg), a polyethylene tube filled with 25% MgSO₄ was inserted into the carotid artery and connected to a pressure transducer (Model P10EZ, Becton Dickinson) for monitoring an arterial pressure. A systolic, a diastolic and an average arterial pressures measured by the pressure transducer were recorded by a recorder being connected to an amplifier (model AP-621, Nihon Kohden Corporation).

After injecting a saline (600 µL) into each test animal for confirming that a blood pressure was stable, a solution of VEGF-like protein (600 µL) was administered from the left femoral vein. After intravenously injecting the VEGF-like protein, its effects on systolic and diastolic blood pressures were calculated as a lowering rate at the maximum blood pressure reduction on the basis of a pressure before the administration. For determining effects of an NO synthetase inhibitor N_{ω}-nitro-L-arginine (L-NNA) in VEGF-like protein induced hypotension, L-NNA (600 µL) was administered from the right femoral vein in a dose of 2.5 µg/g before or after intravenous injection of the VEGF-like protein.

The results obtained from an experiment in which an animal number per group n was at least 3 were expressed as a mean ± SEM. Significance was evaluated by a t-test. When a P value is less than 0.05, the result was determined to be significant.

FIG. 3A shows the results observed in the course of time for change in arterial pressure before and after intravenous injection of vammin in a dose of 0.3 µg/g. The pressure was rapidly lowered immediately after intravenous administration of vammin and the maximum lowering effect was achieved 3 to 5 min after administration. FIG. 3B shows dosage dependency of a rate of systolic and diastolic arterial pressure reduction caused by intravenous administration of vammin. Even intravenous injection of vammin in a dose of 30 ng/g induced a significant blood lowering. Although saturation tendency was observed in a dose of 0.1 µg/g or more, the effect was dose-dependently increased and the maximum blood pressure lowering was observed in a dose of 0.3 µg/g. The dose-response curve in FIG. 3B shows stronger hypotensive effect on a diastolic pressure than on a systolic pressure. Assuming that blood pressure lowering effect in a dose of 0.3 µg/g is 100 %, EC₅₀ values of hypotensive effect on a diastolic and a systolic pressure in intravenous administration of vammin are estimated to be 24 ng/g and 29 ng/g, respectively. VR-1 and HF also showed drastic blood pressure lowering by intravenous administration. For hypotensive effect in intravenous administration, estimated EC₅₀ values of VR-1 and HF based on the dose-response curve are 10 ng/g and 15 ng/g for a diastolic pressure, and 15 ng/g and 18 ng/g for a systolic pressure, respectively.

The maximum pressure lowering was 56.0±6.8 % in a diastolic pressure and 24.7±5.2 % in a systolic pressure for vammin (n=3); 48.4±1.7 % in a diastolic pressure and 16.7±1.1 % in a systolic pressure for VR-1 (n=3); and 52.0±1.9 % in a diastolic pressure and 24±5.9 % in a systolic pressure for HF (n=3). Although among these three venom-derived VEGF-like proteins, VR-1 has slightly lower effect than the other two, these three venom-derived VEGF-like proteins exhibit essentially comparable effect in terms of hypotensive action. The reported values demonstrated that human VEGF shows hypotensive effect as high as an average arterial pressure lowering of about 20 to 25 %, while vammin and VR-1 exhibited hypotensive effects as high as 41 % and 40 % lowering, respectively, as for an average arterial pressure. The venom-derived VEGF-like proteins; vammin, VR-1 and HF show hypotensive effects giving rise to such rapid lowering to a diastolic pressure, which leads to the conclusion that these venom-derived VEGF-like proteins may have influence on peripheral vessels.

Next, for determining whether NO is involved in a mechanism of hypotension induced by these venom-derived VEGF-like proteins, an NO synthetase inhibitor: N_{ω}-nitro-L-arginine (L-NNA), was used to examine whether the inhibitor may block the hypotensive effect or not. After inducing hypotension by intravenous injection of a venom-derived VEGF-like protein, post-administration of L-NNA in the above dose restored a blood pressure to a normal level. Furthermore, pre-administration of L-NNA in the above dose completely inhibited hypotension induction by intravenous injection of a venom-derived VEGF-like protein. However, when administering phenylephrine as an agonist to α-adrenergic receptor, any inhibitory effect on hypotension was not observed. These results strongly demonstrate that NO is involved in hypotensive effect induced by a venom-derived VEGF-like protein and the mechanism . is mediated by activation of an NO synthetase.

### 5. Immunoassay analysis for NO synthetase activation

The immunoassay described below was used for demonstrating that the venom-derived VEGF-like proteins; vammin, VR-1 and HF induce NO synthetase activation by binding to KDR.

Bovine coronary artery endothelial cells (CAECs) were cultured in DMEM (Dulbecco's modified Eagle's medium) containing 10 % fetal bovine serum in a φ10 cm culturing dish. After culturing up to a sub-confluent state (70 to 80 %), the cultured cells were rinsed twice with PBS (phosphate buffered saline) and then cultured in a serum-free DMEM for 15 to 18 hours.

The CAECs cultured in the serum-free DMEM were treated with a solution containing a venom-derived VEGF-like protein (1 nM), quickly washed twice with an ice-cooled PBS, and then collected in 400 µL of Triton/NP-40 lysis buffer (0.5% Triton X-100, 0.5% NP-40, 10 mM Tris-HCl pH 7.5, 2.5 mM KCI, 150 mM NaCl, 30 mM β-glycerophosphate, 50 mM NaF, 1 mM Na₃VO₄, 0.1 % Protease inhibitor cocktail (Sigma)) using a cell scraper.

The cells collected in the lysis buffer were stirred at 4 °C for one hour in a rotator, and the cell debris was centrifuged at 15,000 x g at 4 °C for 15 min, to remove the precipitate (insoluble fraction). A protein concentration in the collected supernatant was determined by bicinchoninic acid protein assay (Pierce, PO, USA). Using an equal amount of the supernatant sample, the proteins contained were subjected to electrophoresis on a 6.5 % polyacrylamide gel to be transferred onto a PVDF film. The total amount of the eNOSs and the amount of phosphorylated eNOS (serine1177) separated by electrophoresis were detected by a Vectastain ABC kit (Vector Laboratories, CA, USA) using an anti-eNOS antibody and an anti-phosphorylated eNOS antibody, respectively. They were visualized by an enhanced chemiluminescence (ECL plus) system (Amersham Bioscience).

FIG. 4 shows the evaluation results detected by immunoblotting that indicate the time-course of changes in the total amount of eNOS proteins (lower) and the amount of the phosphorylated eNOS proteins (upper) being induced in the CAEC cells post to treatment with vammin (1 nM). The vammin (1 nM) treatment induced phosphorylation of serine 1177 in the eNOS proteins in the course of time. The effect reached the maximum after 5 min, and after 60 min, returned to as low level as that of an unstimulated state. Treatment with VR-1 (1 nM) or HF (1 nM) also induced eNOS protein phosphorylation to the comparable level with that for vammin. These results imply that the venom-derived VEGF-like proteins; vammin, VR-1 and HF effect their hypotensive action via NO produced by an NO synthetase activated by phosphorylation of serine 1177.

### 6. Analysis of receptor binding affinity by surface plasmon resonance

Receptor binding for the venom-derived VEGF-like. proteins; vammin, VR-1 and HF was analyzed by means of surface plasmon resonance.

An apparatus for measurement based on surface plasmon resonance technique: BIAcore 3000 (Biosensor) was used to evaluate the properties of binding to and dissociation from a VEGF receptor as for a VEGF-like protein. Experiment for analysis of the receptor binding was conducted at 25 °C using an HBS buffer (10 mM HEPES, pH 7.4, 150 mM NaCl, 3 mM EDTA, 0.005% polysorbate 20). FIt-1-IgG and KDR-IgG were immobilized on a CM5 sensor chip using an amine coupling kit (Biosensor), respectively.

The venom-derived VEGF-like proteins; vammin, VR-1 and HF as well as a recombinant human VEGF165 were diluted with an HBS buffer to prepare solutions with protein concentrations of 3, 10 and 30 nM. They were injected into a flow cell at a flow rate of 20 µL/min. A signal from a blank flow channel as a negative control was subtracted as a background signal from the signal from the sample flow channel, for background correction. In the association process, while injecting a solution with the above protein concentration on the sensor chip at the above flow rate, increase in the resonance signal was detected. In the dissociation process, while injecting the above buffer on the sensor chip at the above flow rate, decrease in the resonance signal was measured.

A rate constant for association (kₐₛₛ), a rate constant for dissociation (k_{diss}) and the maximum binding amount (Rₘₐₓ) for binding to a VEGF receptor in each VEGF-like protein were calculated using a BIA evaluation 3.1 curve fitting software (Biosensor). An apparent dissociation constant (K_{d}) was calculated as a ratio of the rate constants (k_{diss}/kₐₛₛ) from the calculated values.

FIG. 5a shows comparison between the changes in resonance signals measured at the stage (between 100 to 220 sec) of association to and the stage (220 sec or later) of dissociation from a receptor molecule: FIt-1-IgG immobilized on a CM5 sensor chip for human VEGF165 (10 nM; dotted line) and vammin (10 nM; straight line). FIG. 5b shows comparison between the changes in resonance signals measured at the stage (between 100 to 220 sec) of association to and the stage (220 sec or later) of dissociation from a receptor molecule: KDR-IgG immobilized on a CM5 sensor chip for human VEGF165 (30 nM; dotted line) and vammin (30 nM; straight line). FIG. 5c shows comparison between the changes in resonance signals measured at the stage (between 100 to 220 sec) of association to and the stage (220 sec or later) of dissociation from a receptor molecule: Flt-4 (VEGF receptor type 3) immobilized on a CM5 sensor chip for VEGF-C156S variant (500 nM; dotted line) and vammin (500 nM; straight line). FIG. 5d shows comparison between the changes in resonance signals measured at the stage (between 100 to 220 sec) of association to and the stage (220 sec or later) of dissociation from a receptor molecule: neuropilin-1-IgG immobilized on a CM5 sensor chip for human VEGF165 (30 nM; dotted line) and vammin (30 nM; straight line). Human VEGF165 was bound on both Flt-1-IgG and KDR-IgG with higher affinity. On the other hand, vammin was bound to KDR with higher affinity, but did not substantially exhibit binding affinity to FIt-1. Furthermore, Vammin did not substantially exhibit binding affinity to Flt-4 or neuropilin-1-IgG.

Table 1 shows calculated rate constant for association (kₐₛₛ), rate constant for dissociation (k_{diss}) and the maximum binding amount (Rₘₐₓ) as well as an apparent dissociation constant (K_{d}) to each of FIt-1-Ig and KDR-IgG for the venom-derived VEGF-like proteins: vammin, VR-1 and HF as well as the recombinant human VEGF165. Both kₐₛₛ and k_{diss} to KDR for vammin were lower than those for human VEGF165. The results indicate that binding of vammin to KDR is slower and more tight than that of VEGF165.

In quite similar manner to vammin, both VR-1 and HF were bound to KDR with higher affinity, but was not bound to Flt-1. The kₐₛₛ and the k_{diss} for HF indicated strong binding like to that in vammin, while VR-1 exhibited binding property like to that in VEGF165.

**Table 1**

| Kinetic parameters for venom-derived VEGF-like proteins binding to an immobilized VEGF receptor | | | | |
|---|---|---|---|---|
| | kₐₛₛ(M⁻¹·S⁻¹ | k_{diss}(S⁻¹ | K_{d}(M) | Rmax |
| | (×10⁴) | (×10⁻⁶) | (×10⁻¹⁰) | |
| FIt-1-IgG | | | | |
| VEGF165 | 4.29 | 0.46 | 0.11 | 344 |
| vammin | nd^{a} | nd | nd | nd |
| VR-1 | nd | nd | nd | nd |
| HF | nd | nd | nd | nd |

| KDR-IgG | | | | |
|---|---|---|---|---|
| VEGF165 | 4.24 | 11.0 | 2.60 | 316 |
| vammin | 1.12 | 4.6 | 4.11 | 273 |
| VR-1 | 3.90 | 12.9 | 3.32 | 229 |
| HF | 2.88 | 9.6 | 3.34 | 283 |

| | | | | |
|---|---|---|---|---|
| ^{a} nd, no specific binding affinity | | | | |

From the measurement results of the association and the dissociation curves for three levels of protein concentration (3 to 30 nM) in the venom-derived VEGF-like proteins, rate constants for association (kₐₛₛ), rate constants for dissociation (k_{diss}) and the maximum binding amounts (Rₘₐₓ) which allow for optimal curve fitting were calculated using BIA evaluation 3.1. Comparable results were obtained for four independent runs for each measurement. The dissociation constants (K_{d}) listed in the table are estimated values as a ratio of k_{diss}/kₐₛₛ.

### 7. Evaluation of cell growth stimulating activity

Activity for stimulating cell growth was evaluated by cell counting according to the WST-8 method. To 96-well plate were seeded with bovine aortic endothelial cells at 3,000 cells/well. After culturing for 6 hours, the culture medium was replaced with a medium containing 0.1 % fetal bovine serum. After further culturing for 18 hours, vammin and VEGF165 (positive control) were added to the culture, culturing was continued for 6 days, and then cell numbers were counted. An absorption coefficient A₄₉₂₋₅₄₅ of coloring in the WST-8 method was used as an indicator for number of alive cells. FIG. 6a compares measured concentration-dependencies of stimulating activity for proliferation of endothelial cells of vammin (colored column) and human VEGF165 (positive control; white column). Within the tested concentration range of 0.1 to 30 nM, vammin exhibited higher activity than VEGF165 (positive control).

In addition, hypotensive effect was compared between vammin and VEGF165 (positive control) using the method described in the above example 4. FIG. 6b compares the time-course changes in a carotid arterial pressure that were monitored in the course of time before and after intravenous administration in a dose of 0.1 µg/g for vammin or VEGF165 (positive control). FIG. 6c compares the observed maximum reductions in an average arterial pressure (MAP) post to the intravenous administration in a dose of 0.1 µg/g of vammin or VEGF165 (positive control). The results therein are shown as average values for a group consisting of 3 to 4 animals (n=3 to 4). In comparison with VEGF165 (positive control), Vammin shows significantly higher hypotensive effect (P<0.05).

### Industrial Applicability

Vammin and VR-1 can be used, in place of a natural VEGF-A protein, as a hypotensive agent; a therapeutic drug for an ischemic disease utilizing the platelet aggregation inhibitory and anti-thrombogenic activity; a therapeutic drug for vascular endothelial cell damage which is applicable to treatment of a vascular inner wall damaged by percutaneous transluminal coronary intervention. (PCl) being a surgical procedure for arteriosclerosis; or a therapeutic drug for hepatitis in which hepatic cells are to be protected utilizing the antiinflammatory effect, by means of their potent nitrogen monoxide (NO)-dependent hypotensive activity and/or angiogenesis promoting effect induced by binding of the VEGF-like protein to KDR. In these applications, side effects such as excessive proliferation of hepatic cells and hypertrophy of the liver due to binding to Flt-1, which may be concerns during long-term administration, may be significantly reduced.

## Claims

1. A vascular endothelial growth factor (VEGF) like protein derived from *Vipera ammodytes ammodytes;* vammin, wherein the vammin protein has binding activity to a vascular endothelial growth factor receptor type 2 (VEGF receptor 2; KDR), but does not exhibit any binding affinity to a vascular endothelial. growth factor receptor type 1 (VEGF receptor 1; FIt-1), vascular endothelial growth factor receptor type 3 (VEGF receptor 3; FIt-4) or neuropilin-1; and is a homo-dimer where two peptide chain units having the amino acid sequence of SEQ. ID. No. 1 are coupled together via an interchain sulfide bond.

2. A VEGF-like protein variant being a variant protein derived from the vammin protein, wherein
the variant protein is a homo-dimer where two peptide chain units receiving modification by replacing an amino acid contained in the amino acid sequence of SEQ. ID. No. 1 with another amino acid are coupled together via an interchain sulfide bond;
said modified amino acid sequence has variations from that of SEQ. ID. No. 1 such that the 22^{nd} amino acid residue from N-terminus is Ser, the 55^{th} amino acid residues from N-terminus is Arg, and the 74^{th}, 77^{th} and 79^{th} amino acid residues from N-terminus are any one selected from Arg, Ser or Lys, respectively, with the proviso that a choice resulting in that identical to the amino acid sequence of SEQ. ID. No. 3 is excluded therefrom; and
the variant protein has binding activity to a vascular endothelial growth factor receptor type 2 (VEGF receptor 2; KDR), but does not exhibit any binding affinity to a vascular endothelial growth factor receptor type 1 (VEGF receptor 1; FIt-1), vascular endothelial growth factor receptor type 3 (VEGF receptor 3; Flt-4) or neuropilin-1.

3. A vascular endothelial growth factor (VEGF) like protein derived from *Daboia russelli russelli;* VR-1, wherein
the VR-1 protein has binding activity to a vascular endothelial growth factor receptor type 2 (VEGF receptor 2; KDR), but does not exhibit any binding affinity to a vascular endothelial growth factor receptor type 1 (VEGF receptor 1; FIt-1), vascular endothelial growth factor receptor type 3 (VEGF receptor 3; FIt-4) or neuropilin-1; and is a homo-dimer where two peptide chain units having the amino acid sequence of SEQ. ID. No. 2 are coupled together via an interchain sulfide bond.

4. A VEGF-like protein variant being a variant protein derived from VR-1 protein; wherein
the variant protein is a homo-dimer where two peptide chain units receiving modification by replacing an amino acid contained in the amino acid sequence of SEQ. ID. No. 2 with another amino acid are coupled together via an interchain sulfide bond;
said modified amino acid sequence has variations from that of SEQ. ID. No. 2 such that the 55^{th} amino acid residues from N-terminus is Arg, and the 74^{th}, 77^{th} and 79^{th} amino acid residues from N-terminus are any one selected from Arg, Ser or Lys, respectively; and
the variant protein has binding activity to a vascular endothelial growth factor receptor type 2 (VEGF receptor 2; KDR), but does not exhibit any binding affinity to a vascular endothelial growth factor receptor type 1 (VEGF receptor 1; FIt-1), vascular endothelial growth factor receptor type 3 (VEGF receptor 3; FIt-4) or neuropilin-1.

5. A process for purifying and isolating the vammin protein as claimed in Claim 1 from the venom of *Vipera ammodytes ammodytes,* comprising the step of purifying vammin protein included in said venom collected from the snake by means of multi-step chromatography; and
wherein the vammin protein thus isolated shows a band at 25.8 kDa in SDS-PAGE analysis under non-reductive conditions.

6. A process for purifying and isolating the VR-1 protein as claimed in Claim 3 from the venom of *Daboia russelli russelli,* comprising the step of purifying VR-1 protein contained in said venom collected from the snake by means of multi-step chromatography; and
wherein VR-1 protein thus isolated shows a band at 25.2 kDa in SDS-PAGE analysis under non-reductive conditions.

7. Use of the venom-derived VEGF-like protein or variant protein thereof as claimed in any one of Claims 1 to 4 or HF protein having specific binding activity to KDR which is a homo-dimer composed of two peptide chains having the amino acid sequence of SEQ. ID. No. 3, as an active ingredient possessing hypotensive effect for preparing a pharmaceutical composition comprising a hypotensive agent,
wherein said pharmaceutical composition comprises, as the active ingredient possessing hypotensive effect, at least one VEGF-like protein selected from the group consisting of the HF protein which is a homo-dimer consisting of two peptide chains having the amino acid sequence of SEQ. ID. No. 3, the vammin protein as claimed in Claim 1, the vammin protein variant as claimed in Claim 2, the VR-1 protein as claimed in Claim 3 and the VR-1 protein variant as claimed in Claim 4 in combination with a carrier therefor.

8. Use of the venom-derived VEGF-like protein or variant protein thereof as claimed in any one of Claims 1 to 4 or HF protein having specific binding activity to KDR which is a homo-dimer composed of two peptide chains having the amino acid sequence of SEQ. ID. No. 3, as the VEGF-like protein ingredient for preparing a pharmaceutical composition usable for the purpose of treating hepatitis comprising an active ingredient exhibiting effect for inducing KDR-mediated protective action on hepatic cells which utilizes effect of cell proliferation caused by binding of the VEGF-like protein to KDR,
wherein said pharmaceutical composition comprises, as the active ingredient exhibiting effect for inducing KDR-mediated protective action on hepatic cells, at least one VEGF-like protein selected from the group consisting of the HF protein which is a homo-dimer consisting of two peptide chains having the amino acid sequence of SEQ. ID. No. 3, the vammin protein as claimed in Claim 1, the vammin protein variant as claimed in Claim 2, the VR-1 protein as claimed in Claim 3 and the VR-1 protein variant as claimed in Claim 4 in combination with a carrier therefor.

9. Use of the venom-derived VEGF-like protein or variant protein thereof as claimed in any one of Claims 1 to 4 or HF protein having specific binding activity to KDR which is a homo-dimer composed of two peptide chains having the amino acid sequence of SEQ. ID. No. 3, as the VEGF-like protein ingredient for preparing a pharmaceutical composition usable for the purpose of treating vascular endothelial damage associated with percutaneous transluminal coronary intervention (PCI) being one of surgical treatments to arteriosclerosis or of treating an ischemic disease, which composition utilizes vasorelaxation effect, inhibition of platelet aggregation, antithrombotic effect and antiinflammatory effect stimulated by KDR-mediated activation of NO synthetase caused by binding of the VEGF-like protein to KDR,
wherein the pharmaceutical composition comprises, as the VEGF-like protein ingredient possessing binding activity to KDR, at least one VEGF-like protein selected from the group consisting of the HF protein which is a homo-dimer consisting of two peptide chains having the amino acid sequence of SEQ. ID. No. 3, the vammin protein as claimed in Claim 1, the vammin protein variant as claimed in Claim 2, the VR-1 protein as claimed in Claim 3 and the VR-1 protein variant as claimed in Claim 4 in combination with a carrier therefor.

10. Use of a gene or nucleic acid molecule having a nucleotide sequence encoding the venom-derived VEGF-like protein or variant protein thereof as claimed in any one of Claims 1 to 4 or HF protein having specific binding activity to KDR which is a homo-dimer composed of two peptide chains having the amino acid sequence of SEQ. ID. No. 3, as the gene or nucleic acid molecule having a nucleotide sequence encoding the VEGF-like protein, which is contained in the form of a vector for gene recombination where the gene is inserted into a recombinable vector, for recombinational expression of the VEGF-like protein in a host cell from the gene or nucleic acid molecule having a nucleotide sequence encoding a VEGF-like protein having binding activity to KDR,
wherein, as said gene or nucleic acid molecule having a nucleotide sequence encoding the VEGF-like protein, a gene or nucleic acid molecule having a nucleotide sequence encoding at least one VEGF-like protein selected from the group consisting of the HF protein which is a homo-dimer consisting of two peptide chains having the amino acid sequence of SEQ. ID. No. 3, the vammin protein as claimed in Claim 1, the vammin protein variant as claimed in Claim 2, the VR-1 protein as claimed in Claim 3 and the VR-1 protein variant as claimed in Claim 4 is contained in the form of a gene recombination vector where the gene is inserted into the recombinable vector to perform recombinant expression of the VEGF-like protein in the host cell.

11. Use of the venom-derived VEGF-like protein or variant protein thereof as claimed in any one of Claims 1 to 4 or HF protein having specific binding activity to KDR which is a homo-dimer composed of two peptide chains and having the amino acid sequence of SEQ. ID. No. 3, as a VEGF-like protein reagent having binding affinity to the KDR, for the construction of an *in vitro* assay system for inhibitory effect on binding to KDR to screen a compound having inhibitory effect on binding to KDR of the VEGF-like protein possessing binding affinity to KDR,
wherein the *in vitro* assay system for inhibitory effect on binding to KDR is an assay system evaluating blocking activity of a test compound present in the system on the binding by detecting change in the amount bound to KDR as to the VEGF-like protein reagent possessing binding affinity to KDR, and
wherein at least one of the VEGF-like proteins selected from the group consisting of the HF protein which is a homo-dimer composed of two peptide chains having the amino acid sequence of SEQ. ID. No. 3, the vammin protein as claimed in Claim 1, the vammin protein variant as claimed in Claim 2, the VR-1 protein as claimed in Claim 3 and the VR-1 protein variant as claimed in Claim 4 is used as the VEGF-like protein reagent possessing binding affinity to KDR.

12. Use of the venom-derived VEGF-like protein or variant protein thereof as claimed in any of Claims 1 to 4 or HF protein having specific binding activity to KDR which is a homo-dimer composed of two peptide chains having the amino acid sequence of SEQ. ID. No. 3 in preparing a pharmaceutical composition comprising a compound having inhibitory effect on binding affinity to KDR of the VEGF-like protein as an active ingredient for treating an angiogenesis disease,
wherein said pharmaceutical composition comprises, as an active ingredient, a compound having blocking activity on the binding to KDR selected by utilizing the assay system for inhibitory effect on binding to KDR as claimed in Claim 11, in the mechanism for the inhibition of said binding affinity to KDR, which compound is bound on the site for ligand-binding in KDR to block the ligand-binding, or is bound on the site other than the site for ligand-binding in KDR to change the conformation of the binding site, resulting in the prevention of ligand-binding, and
wherein in the selection by using the assay system for inhibitory effect on binding to KDR, at least one of the VEGF-like proteins selected from the group consisting of the HF protein which is a homo-dimer composed of two peptide chains having the amino acid sequence of SEQ. ID. No. 3, the vammin protein as claimed in Claim 1, the vammin protein variant as claimed in Claim 2, the VR-1 protein as claimed in Claim 3 and the VR-1 protein variant as claimed in Claim 4 is used as the VEGF-like protein reagent possessing binding affinity to KDR.

13. The use as claimed in Claim 12, wherein said angiogenesis disease is one of diseases chosen from solid tumor growth, diabetic retinopathy, premature infant retinitis, chronic rheumatoid arthritis or psoriasis.


SEQUENCE LISTING
<110> NEC SOFT CORPORATION & Dr. Takashi MORITA

<120> A VEGF-like protein originated from snake venom having a specific
binding affinity to KDR, and use thereof

<130> NE+04P116

<150> JP 2003-344994
<151> 2003-10-02

<160> 7

<170> MS-WORD

<210> 1
<211> 110
<212> PRT
<213> Vipera ammodytes ammodytes

<220>
<223> Monomeric peptide for homo-dimer formation of vascular endothelial
growth factor like protein: vammin

<400> 1
Glu Val Arg Pro Phe Leu Glu Val His Glu Arg Ser Ala Cys Gln Ala Arg Glu
1               5                   10                  15

Thr Leu Val Pro Ile Leu Gln Glu Tyr Pro Asp Glu Ile Ser Asp Ile Phe Arg
    20                  25                  30                  35
Pro Ser Cys Val Ala Val Leu Arg Cys Ser Gly Cys Cys Thr Asp Glu Ser Leu
            40                  45                  50
Lys Cys Thr Pro Val Gly Lys His Thr Val Asp Leu Gln Ile Met Arg Val Asn
55                  60                  65                  70
Pro Arg Thr Gln Ser Ser Lys Met Glu Val Met Lys Phe Thr Glu His Thr Ala
        75                  80                  85                  90
Cys Glu Cys Arg Pro Arg Arg Lys Gln Gly Glu Pro Asp Gly Pro Lys Glu Lys
                95                  100                 105
Pro Arg
    110

<210> 2
<211> 109
<212> PRT
<213> Daboia russelli russelli

<220> Monomeric peptide for homo-dimer formation of vascular endothelial
growth factor like protein: VR-1
<223>

<400> 2
Glu Val Arg Pro Phe Leu Asp Val Tyr Gln Arg Ser Ala Cys Gln Thr Arg Glu
1               5                   10                  15
Thr Leu Val Ser Ile Leu Gln Glu His Pro Asp Glu Ile Ser Asp Ile Phe Arg
    20                  25                  30                  35

Pro Ser Cys Val Ala Val Leu Arg Cys Ser Gly Cys Cys Thr Asp Glu Ser Met
            40                  45                  50
Lys Cys Thr Pro Val Gly Lys His Thr Ala Asp Ile Gln Ile Met Arg Met Asn
55                  60                  65                  70
Pro Arg Thr His Ser Ser Lys Met Glu Val Met Lys Phe Met Glu His Thr Ala
        75                  80                  85                  90
Cys Glu Cys Arg Pro Arg Trp Lys Gln Gly Glu Pro Glu Gly Pro Lys Glu Pro
                95                  100                 105
Arg

<210> 3
<211> 110
<212> PRT
<213> Vipera aspis aspis

<220>
<223> Monomeric peptide for homo-dimer formation of hypotensive factor: HF

<400> 3
Glu Val Arg Pro Phe Leu Glu Val His Glu Arg Ser Ala Cys Gln Ala Arg Glu
1               5                   10                  15
Thr Leu Val Ser Ile Leu Gln Glu Tyr Pro Asp Glu Ile Ser Asp Ile Phe Arg
    20                  25                  30                  35
Pro Ser Cys Val Ala Val Leu Arg Cys Ser Gly Cys Cys Thr Asp Glu Ser Leu
            40                  45                  50
Lys Cys Thr Pro Val Gly Lys His Thr Val Asp Leu Gln Ile Met Arg Val Asn

55                  60                  65                  70
Pro Arg Thr Gln Ser Ser Lys Met Glu Val Met Lys Phe Thr Glu His Thr Ala
        75                  80                  85                  90
Cys Glu Cys Arg Pro Arg Arg Lys Gln Gly Glu Pro Asp Gly Pro Lys Glu Lys
                95                  100                 105
Pro Arg
    110

<210> 4
<211> 110
<212> PRT
<213> Vipera lebetina

<220>
<223> Monomeric peptide for homo-dimer formation of increasing capillary
permeability protein: ICPP

<400> 4
Glu Val Arg Pro Phe Pro Asp Val His Glu Arg Ser Ala Cys Gln Ala Arg Glu
1               5                   10                  15
Thr Leu Val Ser Ile Leu Gln Glu Tyr Pro Asp Glu Ile Ser Asp Ile Phe Arg
    20                  25                  30                  35
Pro Ser Cys Val Ala Val Leu Arg Cys Ser Gly Cys Cys Thr Asp Glu Ser Leu
            40                  45                  50
Lys Cys Thr Pro Val Gly Lys His Thr Val Asp Met Gln Ile Met Arg Val Asn
55                  60                  65                  70
Pro Arg Thr Gln Ser Ser Lys Met Glu Val Met Lys Phe Thr Glu His Thr Ala

        75                  80                  85                  90
Cys Glu Cys Arg Pro Arg Arg Lys Gln Gly Glu Pro Asp Gly Pro Lys Glu Lys
                95                  100                 105
Pro Arg
    110

<210> 5
<211> 110
<212> PRT
<213> Artificial sequence

<220>
<223> Monomeric peptide for homo-dimer formation of vammin variant having
a mutation of Lys 55 → Arg

<400> 5
Glu Val Arg Pro Phe Leu Glu Val His Glu Arg Ser Ala Cys Gln Ala Arg Glu
1               5                   10                  15
Thr Leu Val Pro Ile Leu Gln Glu Tyr Pro Asp Glu Ile Ser Asp Ile Phe Arg
    20                  25                  30                  35
Pro Ser Cys Val Ala Val Leu Arg Cys Ser Gly Cys Cys Thr Asp Glu Ser Leu
            40                  45                  50
Arg Cys Thr Pro Val Gly Lys His Thr Val Asp Leu Gln Ile Met Arg Val Asn
55                  60                  65                  70
Pro Arg Thr Gln Ser Ser Lys Met Glu Val Met Lys Phe Thr Glu His Thr Ala
        75                  80                  85                  90
Cys Glu Cys Arg Pro Arg Arg Lys Gln Gly Glu Pro Asp Gly Pro Lys Glu Lys

                95                  100                 105

Pro Arg

    110

<210> 6

<211> 109

<212> PRT

<213> Daboia russelli russelli

<220> Monomeric peptide for homo-dimer formation of VR-1 variant having a

mutation of Lys 55 → Arg

<223>

<400> 6

Glu Val Arg Pro Phe Leu Asp Val Tyr Gln Arg Ser Ala Cys Gln Thr Arg Glu

1               5                   10                  15

Thr Leu Val Ser Ile Leu Gln Glu His Pro Asp Glu Ile Ser Asp Ile Phe Arg

    20                  25                  30                  35

Pro Ser Cys Val Ala Val Leu Arg Cys Ser Gly Cys Cys Thr Asp Glu Ser Met

            40                  45                  50

Arg Cys Thr Pro Val Gly Lys His Thr Ala Asp Ile Gln Ile Met Arg Met Asn

55                  60                  65                  70

Pro Arg Thr His Ser Ser Lys Met Glu Val Met Lys Phe Met Glu His Thr Ala

        75                  80                  85                  90

Cys Glu Cys Arg Pro Arg Trp Lys Gln Gly Glu Pro Glu Gly Pro Lys Glu Pro

                95                  100                 105

Arg

<210> 7

<211> 109

<212> PRT

<213> Daboia russelli russelli

<220> Monomeric peptide for homo-dimer formation of VR-1 variant having a

mutation of Ser22 → Pro

<223>

<400> 7

Glu Val Arg Pro Phe Leu Asp Val Tyr Gln Arg Ser Ala Cys Gln Thr Arg Glu

1               5                   10                  15

Thr Leu Val Pro Ile Leu Gln Glu His Pro Asp Glu Ile Ser Asp Ile Phe Arg

    20                  25                  30                  35

Pro Ser Cys Val Ala Val Leu Arg Cys Ser Gly Cys Cys Thr Asp Glu Ser Met

            40                  45                  50

Lys Cys Thr Pro Val Gly Lys His Thr Ala Asp Ile Gln Ile Met Arg Met Asn

55                  60                  65                  70

Pro Arg Thr His Ser Ser Lys Met Glu Val Met Lys Phe Met Glu His Thr Ala

        75                  80                  85                  90

Cys Glu Cys Arg Pro Arg Trp Lys Gln Gly Glu Pro Glu Gly Pro Lys Glu Pro

                95                  100                 105

Arg